# EUROPEAN PATENT SPECIFICATION

(11) **EP 1 762 235 B1**
(45) Date of publication and mention of the grant of the patent: **05.08.2009**
(21) Application number: 06006331.0
(22) Date of filing: 28.03.2006
(51) Int. Cl.: A61K 31/122, A61K 9/08, A61K 47/44, A61P 7/04

(54) **Orally adminstered composition for preventing neonatal hemorrhages, in a tardy form, caused by a K-vitamin deficiency**
Oral zu verabreichende Zubereitung zur Vermeidung von neonatalen Blutungen in einer späten Form, verursacht von einem Vitamin K Mangel
Composition orale pour empêcher des hemorrhagies neonatales en forme tardive, causé par une déficience de vitamine K.

(30) Priority: 08.09.2005 IT CT20050012
(43) Date of publication of application: 14.03.2007
(73) Proprietor: Ammirante, Rosario, 95024 Acireale CT (IT); Molinari, Felice Piero, 20144 Milano (IT)
(72) Inventor: Ammirante, Rosario, 95024 Acireale CT (IT); Molinari, Felice Piero, 20144 Milano (IT)
(74) Representative: Bottero, Carlo

(56) References cited:
- WO-A-02/01969
- WARIYAR U ET AL: "Six years' experience of prophylactic oral vitamin K." ARCHIVES OF DISEASE IN CHILDHOOD. FETAL AND NEONATAL EDITION. JAN 2000, vol. 82, no. 1, January 2000 (2000-01), pages F64-F68, XP009075858 ISSN: 1359-2998
- CAUSA P ET AL: "Prevention of late hemorrhagic disease of newborn with vitamin K" QUADERNI ACP 2005 ITALY, vol. 12, no. 1, 2005, pages 29-30, XP009075877

## Description

### BACKGROUND OF THE INVENTION

The present invention relates to an orally administered compound, for preventing neonatal hemorrhages, in a tardy form, caused by a K-vitamin deficiency.

As is known, from a lot of clinical studies, it has been found that a deficiency of K-vitamin can cause a serious pathology, including a hemorrhagic disease, due to a deficiency of K-vitamin, which can occur either during or after the neonatal period.

Healthy newborn babies are affected by a decreasing of K-vitamin coagulation factor plasmatic concentration, which occurs for some days after the birth, i.e. for a time necessary for receiving a suitable vitamin feeding associated to the diet and to form a regular intestinal bacterial flora.

A hemorrhagic disease due to a deficiency of K-vitamin is caused by an unsuitable activity of the K-dependant factors, such as:
factor II- a prothrombin hepatic biosynthesis
factor VII- a proconvertin hepatic biosynthesis
factor IX - the Chrismas factor - PTC - which is a plasmatic thromboplastinic component.

Factor X - the Stuart-Prower factor, which is related to a K-vitamin deficiency.

The hemorrhagic disease of a newborn baby can be classified according to the newborn baby age, in a precocious form, in a case in which it occurs within the first twenty four hours, in a classic form, with manifestations between the first and seventeenth days, and in a tardy form, as it occurs between the second and thirteenth week/six months and, depending on etiology, in an idiomatic form in breast-fed newborn babies, or in a secondary form deriving from a defective absorption or liver diseases.

The precocious form occurs in a rate from 6 to 12% and is due to the placentary transport of drugs having an inhibitory effect on the K-vitamin effect, such as anti-convulsant, antibiotic, anti-tubercular, anti-coagulating substances.

The classic form occurs in a rate of 0.4 to 1.7%, and in the first life weeks.

The tardy form of the hemorrhagic disease, due to a K-vitamin deficiency, primarily occurs in breast-fed newborn babies, if they are not properly subjected to a related prophylaxis.

In particular, the breast-fed newborn babies represent a high-risk category, since human milk has a low K-vitamin concentration (2 micrograms/liter and 50 micrograms/liter in a matched milk).

Moreover, the intestinal flora of breast-fed newborn babies, which is mainly constituted by lactobacillus acidofilus, does not contain microorganisms adapted to synthetize it.

Thus, the K-vitamin amount or rate derived from the mother placentary transport is quickly eliminated because of its short half-life.

The rate of the tardy disease, in newborn babies not subjected to a proper prophylaxis, varies from 4.4 to 7.2/100,000 vital newborn babies.

An administration of a milligram of K-vitamin by an intramuscular administration way, at the birth, is adapted to prevent a possible hemorrhagic risk of the healthy newborn baby.

However, such an administration does not fully solve the problem with respect to a prophylaxis of the tardy form.

Thus, for the above mentioned reasons, a lot of Countries have chosen to adopt an oral prophylaxis, with different administering protocols.

The administering protocol and way, providing to administer oral doses from a first dose administered at the birth, for preventing the tardy hemorrhagic disease, involve, on the other hand, lacks of homogeneity and a differently interpreted performance.

From available data, it has been found that an administration of repeated oral doses of 25 micrograms/day of K-vitamin to breast-fed newborn babies, was efficient for a full prophylaxis, to reduce and practically fully eliminating the risk of a tardy hemorrhagic disease.

In this connection it should be pointed out that the human need of K-vitamin is very small, and that the intestinal absorption mechanism of K-vitamin compounds, depends on their solubility.

Phytonadion (Kl vitamin) is nearly fully absorbed through the gastrointestinal tract, only if a suitable amount of biliar salts is present, through the lymphatic ways, in the proximal portions of the small intestine.

After the absorption, it is at the start concentrated in the liver, and then the concentration quickly decreases.

Only a small amount of K-vitamin is accumulated in the other tissues, since it is quickly metabolized to higher polar metabolites and excreted through the bile and urine.

The K-vitamin synthesis is also performed by the bacterial flora present in the colon, which, as stated, in a newborn baby is practically absent.

A quick turnover of K-vitamin in a human person has been also demonstrated, since said K-vitamin is practically absent in blood after 72 hours.

In a newborn baby, a blood and liver tissue concentration of K1 vitamin is low, with respect to the values of an adult person, whereas the K2 vitamin, which represent the major component of an adult person liver, is absent.

The hepatic reserve of a newborn baby constitutes about a fifth of that of a grown-up, which exclusively comprises K1 vitamin and, because of the particular dietetics restriction conditions determined by the administration of mother milk only or because of an unsuitable supplement, is quickly drained.

At present, all the pharmaceutical, para-pharmaceutical, dietetics or supplementing compounds, as available on the market, are not suitable for a pediatric and neonatal use, since, for a unitary dose administration only K-vitamin megadoses are actually provided.

An example of a composition known in the art is described in Wariyar et al. "Six years' experience of prophylactic oral vitamin K"; Archive of Disease in Childwood. Fetal and Neonatal Edition. Jan. 2000, vol. 82 no. 1, January 2000, pages F64-F68.
This publication discloses a composition of vitamin K1 dissolved in medium chain triglyceride oil.

Moreover, for an oral administration, in the form of masticable pills or tablets, excipients and adjuvants such as titanium bioxide, caramel, glycerol, ethylvanillin, paraffins, saccharose, colloidal silica, cocoa butter and so on are used.

When formulated as drops K-vitamin is associated with excipients, such as a ricinoleic polyethyleneglycol derivate, benzoic acid, methyl-p-hydroxybenzoate, propyl-p-hydroxybenzoate.

For example, Rote Liste 2002, page 16, no. 007 (Konaklon^{®}) describes aqueous based compositions of vitamin Kl that comprise other additives: phenol in one and glycocholic acids in the other two.

The formulations presently available on the market do not allow easy performance of a fractionation of the megadose for therapeutical needs.

Actually, it is practically very difficult to provide an administration of a proper amount of the active principle.

Moreover, processing adjuvants are very objectable, because of the synthetic nature of the used materials and since they are extraneous with respect to the vitamin.

Thus, small amounts of chemical aids, the so-called preserving substances designed for operating as stabilizer or excipient agents, are administered to the newborn baby.

On the other hand, said latter substances, in addition to the fact that they cannot be properly used in a pediatric neonatal application, because of recently adopted European sanitary rules and since they will be presumably put aside in the immediate future, or anyhow strictly regulated.

### SUMMARY OF THE INVENTION

Accordingly, the aim of the present invention is to solve the above mentioned problems, by providing a formulation which can be orally administered in a prophylaxis protocol for preventing neonatal hemorrhages, in their tardy form, caused by a K-vitamin deficiency, which can be easily metered. Said formulation is obtained in liquid form suitable for a delivery in the form of a number of compound drops which can be easily preset.

An object of the invention is to provide an orally administerable formulation for use in preventing neonatal hemorrhages in a tardy form caused by a K-vitamin deficiency, **characterized in that** said formulation consists of a K1 vitamin dissolved in a vegetable oil.

A further object of the invention is to provide a method for preparing the above formulation, wherein said formulation is made by mixing increasing doses of an oil solvent and K1 vitamin in a dissolving mixing device.

Within the scope of the above mentioned aim, a main object of the invention is to provide formulation in which the solvent vector is also adapted to provide a protective action on the active principle, by properly embedding it without any element decanting or separation.

Another object of the present invention is to provide a formulation which, owing to its specifically designed features, is very reliable and safe in operation.

Yet another object of the present invention is to provide a formulation which can be easily made by using easily available elements and materials and which, moreover, is very competitive from a mere economic standpoint.

According to one aspect of the present invention, the above mentioned aim and objects, as well as yet other objects, which will become more apparent hereinafter, are achieved by an orally administered formulation for preventing neonatal hemorrhages in a tardy form, caused by a K-vitamin deficiency, characterized in that said formulation comprises K1 vitamin, in an oil solution form, suitable for an administration by daily microdoses.

Further characteristics and advantages of the present invention will become more apparent hereinafter from the following detailed disclosure of the orally administered formulation for preventing neonatal hemorrhages, in a tardy form, caused by a K-vitamin deficiency, according to the invention.

Said formulation comprises a natural carrier, i.e. a solvent vector, including a vegetal oil; the dissolution of the vitamin active principle in oil is perfect since K1 vitamin is liposoluble.

The oil solvent provides an active principle protective function, since it suitably embeds said active principle therein, without causing any decanting or separating of the elements.

Moreover, a proper absorption of K1 vitamin is facilitated by the presence of fat substances, and in particular oils, which constitute simple fat materials which, since they are water insoluble biomolecules, provide a very important cellular energy source.

A main aspect of the present invention is that the inventive formulation does not include water, thereby in addition to providing an improved preserving, it also provides a low risk of microbiological polluting.

Yet another important aspect is that the inventive formulation does not include preservatives and coloring substances.

In fact, the formulation provides the use of K1 vitamin and a vegetal oil, which comprises all the naturally occurring vegetal oils, or defined as such by the enforcing legal rules.

Moreover, the formulation has a fully neutral taste, thereby it is not necessary to add flavoring substances to said formulation.

Moreover, the formulation can be administered in a drop form, to prevent excessive doses from being administered, as caused by an improper delivery of an excessive amount of drops, with respect to the prescribed drop number.

In particular, the formulation is made by mixing, in an increasing dose rate manner, the two compound elements in a suitable dissolving or mixing device.

In preparing the compound, the mixed substances are disaerated to eliminate possible air bubbles embedded in the oil , and then the mixture is packaged in pharmaceutical dark glass vessels, closed by suitable closing plugs.

A further advantage of the product is that, in a primary preparing step thereof, i.e. as the oil is filled in vessels, any air present in the vessel inside is eliminated, thereby increasing the preserving period, also due to the presence of a suitable stopper or plugging arrangement.

From the above disclosure it should be apparent that the invention fully achieves the intended aim and objects.

In particular, the inventive formulation can be easily metered, since the K1 vitamin is dissolved in an oil vector, thereby optimizing all the absorption factors of the formulation.

The invention, as disclosed, is susceptible to several modifications and variations, all coming within the inventive scope.

Moreover, all the details can be replaced by other technically equivalent elements.

## Claims

1. An orally administerable formulation for use in preventing neonatal hemorrhages in a tardy form caused by a K-vitamin deficiency, **characterized in that** said formulation consists of a K1 vitamin dissolved in a vegetable soil.

2. The formulation according to the preceding claim, which is in liquid form for a delivery in the form of drops.

3. A method for preparing the formulation according to claim 1 or 2, wherein said formulation is made by mixing increasing doses of an oil solvent and Kl vitamin in a dissolving mixing device.

4. The method according to claim 3, wherein said formulation is subjected to a disaerating step to remove therefrom air embedded in the oil solvent.

5. The method according to claim 3 or 4, wherein said formulation is adapted to be preserved in dark glass vessels.

6. The formulation according to claim 1 or 2, wherein said formulation is an under-vacuum packaged formulation

## Patentansprüche

1. Oral verabreichbare Formulierung zur Verwendung zur Vermeidung von neonatalen Blutungen in einer späten Form, die von einem Mangel an Vitamin K verursacht werden, **dadurch gekennzeichnet, daß** die benannte Formulierung aus einem in einem Pflanzenöl gelösten Vitamin Kl besteht.

2. Formulierung nach dem vorherigen Anspruch, die in flüssiger Form für die Abgabe als Tropfen liegt.

3. Verfahren zur Herstellung der Formulierung nach Anspruch 1 oder 2, worin die benannte Formulierung hergestellt wird, indem zunehmende Dosen eines Öl-Lösungsmittels und Vitamins Kl in einer Lösmischvorrichtung vermischt werden.

4. Verfahren nach Anspruch 3, worin die benannte Formulierung einem Entgasungsschritt unterworfen wird, um die im Öl-Lösungsmittel eingearbeitete Luft davon zu entfernen.

5. Verfahren nach Anspruch 3 oder 4, worin die benannte Formulierung zur Lagerung in Behältern aus Dunkelglas geeignet ist.

6. Formulierung nach Anspruch 1 oder 2, worin die benannte Formulierung eine vakuumverpackte Formulierung ist.

## Revendications

1. Formulation pour administration orale pour l'emploi afin d'empêcher des hémorragies néonatales en forme tardive causées par une insuffisance de vitamine K, **caractérisée en ce que** ladite formulation comprend une vitamine K1 dissoute dans une huile végétale.

2. Formulation selon la revendication précédente, en forme liquide pour la délivrance sous forme de gouttes.

3. Procédé pour la préparation de la formulation selon la revendication 1 ou 2, où ladite formulation est obtenue en mélangeant des doses croissantes d'un solvant huileux et de vitamine K1 dans un dispositif mélangeur de dissolution.

4. Procédé selon la revendication 3, où ladite formulation est soumise à une étape de désaération pour éliminer l'air contenu dans le solvant huileux.

5. Procédé selon la revendication 3 ou 4, où ladite formulation est apte à être conservée dans des récipients en verre foncé.

6. Formulation selon la revendication 1 ou 2, où ladite formulation est une formulation emballée sous vide.
